# FASCICULE DE BREVET EUROPEEN

(11) **EP 1 153 581 B1**
(45) Date de publication et mention de la délivrance du brevet: **14.07.2004**
(21) Numéro de dépôt: 00109786.4
(22) Date de dépôt: 09.05.2000
(51) Int. Cl.: A61F 2/06

(54) **Implant vasculaire comprenant un déflecteur central**
Mit einem zentralen Deflektor versehenes vaskuläres Implantat
Vascular implant comprising a central deflector

(43) Date de publication de la demande: 14.11.2001
(73) Titulaire: EndoArt S.A., 1278 La Rippe (CH)
(72) Inventeur: Imbert, Christian, 1278 La Rippe (CH)
(74) Mandataire: Micheli & Cie

(56) Documents cités:
- WO-A-98/58599
- WO-A-99/09911
- WO-A-99/25252

## Description

La présente invention concerne un implant vasculaire et plus particulièrement un implant intra vasculaire comprenant un corps central permettant une action hémodynamique sur la paroi vasculaire et des moyens mécaniques de maintien de la paroi vasculaire. Des tels implants à déflecteur central sont décrits dans la demande de brevet internationale publiée sous le numéro WO 98/58599.

Brièvement résumé, ces implants sont destinés au traitement des rétrécissements ou sténoses des vaisseaux sanguins par angioplastie transluminale. Ils comprennent un corps central maintenu au centre de l'artère par des moyens de maintien qui prennent appui en position de service contre la paroi vasculaire. Le déflecteur central dévie radialement le flux sanguin en direction des parois du vaisseau, ce qui permet d'augmenter localement la contrainte de cisaillement sur la paroi de l'artère ou du vaisseau. Grâce à cette action hémodynamique, on a observé que les sténoses se reforment moins fréquemment que lors de l'utilisation de stents traditionnels qui n'ont qu'une action mécanique de support ou de maintien de la paroi artérielle.

Ces implants à déflecteur présentent toutefois un inconvénient dans le cas où par exemple une nouvelle intervention due à une lésion distale dans le conduit vasculaire doit être envisagée. En effet la présence du déflecteur au centre du conduit vasculaire gêne ou rend impossible la progression d'un cathéter au-delà de l'implant.

L'implant intra vasculaire objet de la présente invention a pour but de remédier à cet inconvénient en proposant un implant qui se distingue par les caractéristiques énumérées à la revendication 1.

Grâce à ces caractéristiques, les implants selon l'invention ont une action différentielle en fonction du temps. Ils permettent de bénéficier de l'action hémodynamique grâce à la présence du déflecteur de flux central durant la période de cicatrisation de la lésion (environ deux à trois mois) puis dans un second temps le déflecteur central se résorbe et enfin les moyens de maintien du déflecteur au centre du conduit reviennent en position contre la surface interne du conduit, libérant de ce fait le passage au centre du conduit.

D'autres avantages ressortent des caractéristiques énoncées dans les revendications dépendantes et de la description qui suit et dans laquelle il est fait référence aux dessins annexés qui représentent schématiquement et à titre d'exemple non limitatif une forme d'exécution d'un implant intra vasculaire selon l'invention.

La figure 1 est une vue de dessus illustrant les découpes pratiquées dans un cylindre de matière représenté déplié.

La figure 2 est une vue schématique en perspective éclatée d'un implant selon l'invention sans le déflecteur central.

La figure 3 est une vue schématique en perspective éclatée d'un implant selon l'invention muni du déflecteur central.

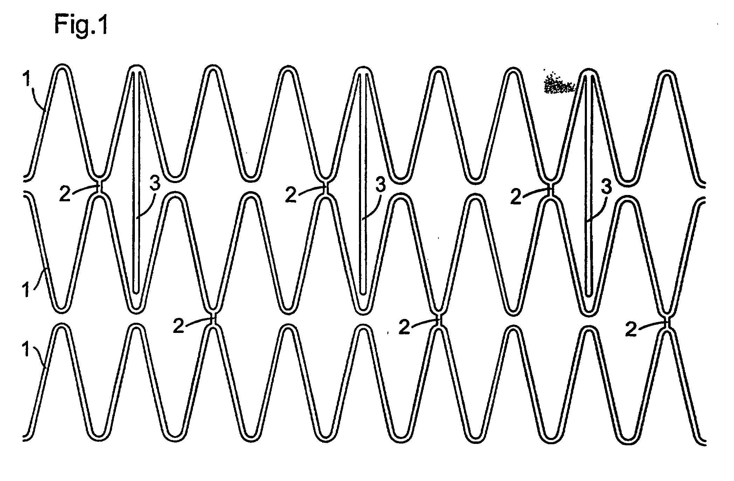

L'implant selon l'invention va maintenant être décrit en référence à la figure 1 qui représente la forme de la découpe pratiquée dans un cylindre creux de matière, ce dernier étant représenté comme si on l'avait ouvert longitudinalement, puis déroulé et mis à plat.

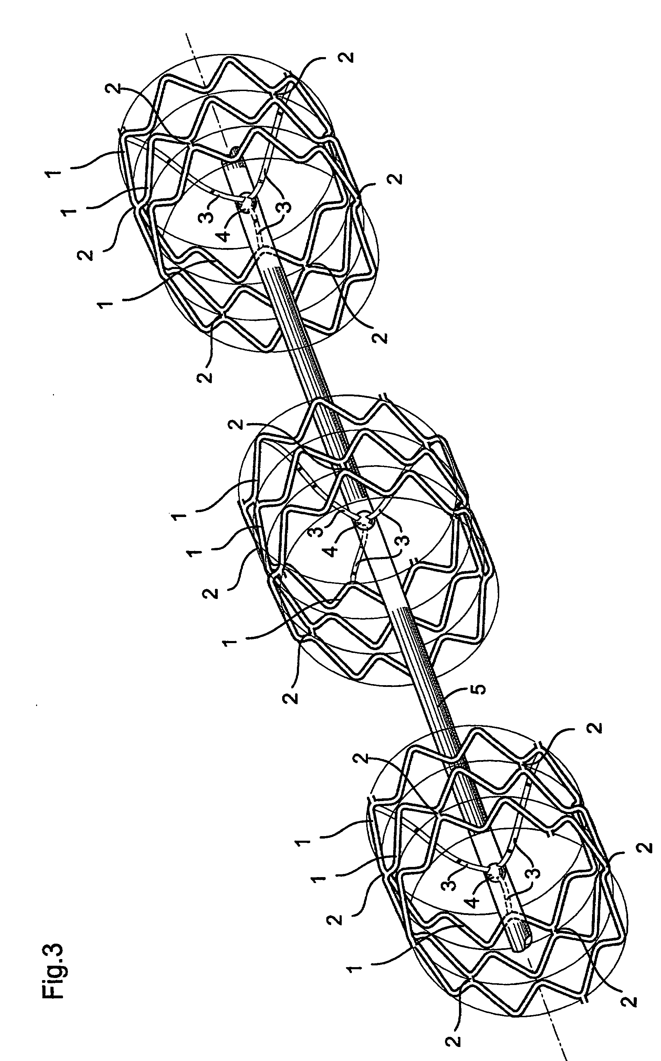

La découpe est réalisée dans le cylindre de manière à former une série de tronçons 1 présentant une configuration en zigzag, chaque série de tronçons en zigzag 1 étant reliée à la série de tronçons adjacente par un ou plusieurs ponts 2 répartis sur la circonférence du cylindre. Le nombre de ponts prévus dépend de la souplesse axiale que l'on désire donner à l'implant, dans l'exemple représenté chaque série de tronçons en zigzag 1 est reliée à la suivante par l'intermédiaire de trois ponts répartis à 120 degrés sur la circonférence du cylindre. Trois bras 3 longitudinaux sont également découpés dans le cylindre. Chacun des bras 3 est solidaire par l'une de ses extrémités d'un tronçon en zigzag 1 et libre à son autre extrémité. Ces bras 3 servent d'organe de maintien et de centrage du déflecteur qui est visible sur la figure 3. On notera que le nombre de bras 3 peut différer dans d'autres formes d'exécution. On peut se contenter de deux bras de maintien 3 ou au contraire en prévoir un nombre plus important, de 4 à 8 par exemple, répartis sur la circonférence du cylindre.

Une fois la découpe réalisée, l'élément obtenu subit un traitement thermique permettant de fixer sa forme à son diamètre maximum qui est légèrement supérieur au diamètre du conduit vasculaire dans lequel il sera implanté.

Pour fabriquer cet élément, on utilise des matériaux à durcissement structural fortement alliés au cobalt dont la structure moléculaire change après un traitement thermique adéquat conférant ainsi à la matière une caractéristique ressort incomparable.

A titre d'exemple non limitatif, des matériaux tels que le Phynox sont parfaitement adaptés. Dans le cas du Phynox, le traitement thermique s'effectue de préférence à une température comprise entre 480 et 550 degrés Celsius et pendant une durée comprise entre 3 et 4 heures.

D'autres matériaux à mémoire comme le Nitinol peuvent également être utilisés pour réaliser de tels implants. Dans ce cas, le traitement thermique dure de 3 à 4 minutes à une température d'environ 600 degrés Celsius.

Deux modes de fabrication sont envisageables pour réaliser l'élément stent de l'implant. Le premier consiste à découper un cylindre creux de matière dont le diamètre correspond à celui de l'implant lorsqu'il est précontraint radialement dans le cathéter qui servira à l'implanter, puis à le déformer mécaniquement à l'aide d'un outil de formage, pour l'ouvrir à son diamètre maximum. Le traitement thermique est appliqué à l'élément ouvert à son diamètre maximum pour en fixer la forme.

Le second mode de fabrication prévoit de découper un cylindre de matière dont le diamètre est légèrement supérieur au diamètre du conduit dans lequel il doit être implanté puis d'en fixer sa forme par traitement thermique.

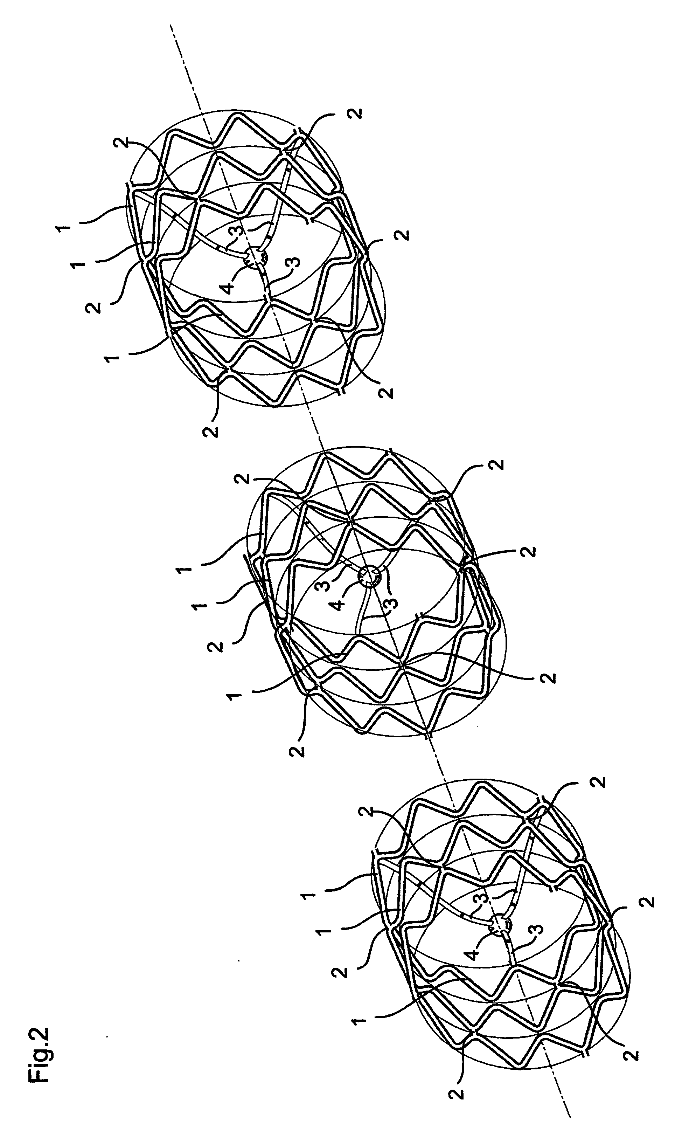

Pour améliorer la clarté des figures 2 et 3, l'implant a été représenté en perspective en trois segments identiques éclatés le long de l'axe longitudinal.

Après la fixation de la forme par traitement thermique, les bras 3 sont contraints vers le centre de la structure cylindrique de l'implant par un outillage spécialement conçu et liés par une goutte 4 de polymère bio-résorbable à dégradation lente (temps de dégradation de l'ordre de 3 mois par exemple).

Les polymères bio-résorbables regroupent plusieurs familles de polymères, comme les polyactides ou les polyglycolides qui se résorbent, dans les conditions physiologiques normales, en produits de dégradation non toxiques aisément métabolisés et éliminés par l'organisme. En fonction de leur composition ces polymères présentent des propriétés mécaniques et des vitesses de dégradation variables et prévisibles.

Lorsque les bras sont solidarisés ensemble à l'aide de la goutte 4, l'implant est placé dans un moule d'injection qui permet d'injecter le déflecteur 5 au centre de la structure, enrobant les gouttes de liaison 4 des bras 3.

Le polymère injecté pour réaliser le déflecteur peut être identique ou différent de celui utilisé pour lier les bras de liaison 3. Lorsque l'on utilise le même polymère pour les gouttes de liaison 4 que pour le déflecteur 5, les gouttes de liaison 4 ne commencent à se dégrader qu'une fois que le déflecteur 5 est complètement résorbé. En effet tant que le déflecteur n'est pas résorbé, les gouttes de liaison 4 ne sont pas soumises au forces de frottement générées par la circulation du fluide dans le conduit puisque enrobées par le déflecteur 5.

Si l'on choisit des polymères présentant des vitesses de dégradation différentes pour les gouttes de liaison 4 et le déflecteur central, on choisira de préférence, pour le déflecteur, un polymère qui présente une vitesse de dégradation ou de résorption supérieure à celle du polymère utilisé pour lier les bras 3. A titre d'exemple, si le temps de dégradation du polymère utilisé pour lier les bras est de l'ordre de trois mois, on utilisera un polymère dont le temps de dégradation est de l'ordre de deux mois pour le déflecteur.

L'implant résultant obtenu par le procédé précédemment décrit et illustré à la figure 3 présente une structure auto extensible comprenant plusieurs séries de tronçons en zigzag 1, reliées entre elles par des ponts 2, qui prennent appui en position de service contre la paroi interne du conduit vasculaire. Pour être mis en place, on contraint radialement l'implant dans un cathéter qui permet de l'acheminer jusqu'à l'endroit de la lésion du vaisseau sanguin ou de l'artère. Lorsque l'implant est libéré dudit cathéter il reprend la forme fixée par le traitement thermique et les tronçons en zigzag 1 exercent une action mécanique de maintien sur la paroi vasculaire. Durant une première période correspondant au temps de dégradation du déflecteur central, l'implant exerce une double action sur le conduit vasculaire; une première action hémodynamique résultant de la déviation du flux sanguin en direction de la paroi du conduit vasculaire grâce à la présence du déflecteur central 5 et une action mécanique de maintien grâce à la structure en zigzag. A l'issue de cette première période, le déflecteur est complètement résorbé et seule l'action de maintien est assurée par la structure en zigzag. Après une seconde période, les gouttes de polymère 4 liant les bras 3 de stabilisation du déflecteur 5 se dégradent et libèrent les bras 3.

De par leur élasticité et pour reprendre leur forme, les bras 3 viennent se plaquer contre la paroi du conduit vasculaire, libérant ainsi le passage au centre du conduit tout en renforçant l'action mécanique de maintien de la paroi vasculaire.

Les implants selon l'invention permettent de favoriser la cicatrisation de la lésion grâce à l'action hémodynamique tout en offrant un soutien adéquat du conduit vasculaire grâce à leur structure. L'action hémodynamique n'est plus nécessaire lorsque la lésion est cicatrisée de sorte que l'on choisira, pour le déflecteur 5, un polymère dont le temps de résorption correspond environ à la période de cicatrisation d'une lésion.

Pour augmenter la sécurité et éviter que le déflecteur 5 ne soit libéré intempestivement alors qu'il n'est pas encore complètement résorbé, par exemple dans le cas d'une dégradation non uniforme du déflecteur 5, on utilisera de préférence, pour la goutte 4 de liaison des bras 3, un polymère dont le temps de résorption est supérieur d'environ un mois à celui du polymère utilisé pour réaliser le déflecteur central 5.

Après dégradation du déflecteur 5 et lorsque les bras 3 sont libérés, une intervention sur une lésion distale peut être réalisée car le conduit vasculaire est libre et autorise facilement le passage de matériel médical au-delà de l'implant.

Plusieurs variantes de réalisation peuvent être envisagées, notamment en ce qui concerne la configuration de la structure en zigzag, qui peut être adaptée en fonction du type de vaisseaux à traiter sans sortir du cadre de l'invention telle que revendiquée.

## Revendications

1. Implant vasculaire comprenant un déflecteur de flux (5) en matériau bio résorbable permettant de dévier les lignes de courant radialement en direction des parois artérielles, et des moyens de maintien (3) du déflecteur (5) au centre du conduit vasculaire, **caractérisé par le fait que** les moyens de maintien du déflecteur sont précontraints au centre de la structure cylindrique et reliés ensemble par l'intermédiaire de moyens bio résorbables.

2. Implant vasculaire selon la revendication 1, **caractérisé par le fait que** les moyens bio résorbables reliant les moyens de maintien du déflecteur (5) présentent une vitesse de dégradation inférieure à la vitesse de dégradation du déflecteur central (5).

3. Implant vasculaire selon l'une des revendications précédentes, **caractérisé par le fait que** les moyens de maintien du déflecteur sont constitués d'une série de tronçons en zigzag (1) reliés entre eux par l'intermédiaire de ponts (2); **par le fait qu'**au moins deux bras de maintien (3), solidaires à l'une de leur extrémité d'un tronçon en zigzag (1) et libres à leur extrémité opposée, sont reliés entre eux par leur extrémité libre à l'aide d'une goutte (4) en polymère bio résorbable.

4. Implant vasculaire selon l'une des revendications précédentes, **caractérisé par le fait qu'**il est obtenu par découpage d'un cylindre de matière creux en un matériau à durcissement structural de manière à dégager au moins deux séries de tronçons en zigzag (1) et au moins deux bras de maintien (3) du déflecteur (5) et **par le fait que** l'élément ainsi découpé est ouvert à son diamètre maximal à l'aide d'un outil de formage puis subit un traitement thermique permettant de fixer sa forme.

5. Implant vasculaire selon l'une des revendications 1 à 3, **caractérisé par le fait qu'**il est obtenu par découpage d'un cylindre de matière creux en un matériau à durcissement structural, présentant un diamètre sensiblement supérieur à celui du vaisseau dans lequel il doit être introduit, de manière à dégager au moins deux séries de tronçons en zigzag (1) et au moins deux bras de maintien (3) du déflecteur (5) et **par le fait que** l'élément ainsi découpé subit un traitement thermique permettant de fixer sa forme.

6. Implant selon l'une des revendications 1 à 3, **caractérisé par le fait que** les moyens de maintien (1,2,3) du déflecteur central (5) sont réalisés dans un matériau à mémoire.

## Patentansprüche

1. Vaskuläres Implantat mit einem Strömungsdeflektor (5) aus bioresorbierbarem Material, der es gestattet, die Strömungslinien radial in Richtung auf die Arterienwandungen abzulenken, sowie Mitteln (3), um den Deflektor (5) in der Mitte des vaskulären Kanals zu halten, **dadurch gekennzeichnet, dass** die Mittel zum Halten des Deflektors zur Mitte der zylindrischen Struktur hin vorgespannt und durch bioresorbierbare Mittel miteinander verbunden sind.

2. Vaskuläres Implantat nach Anspruch 1, **dadurch gekennzeichnet, dass** die bioresorbierbaren Mittel, die die Mittel zum Halten des Deflektors (5) miteinander verbinden, eine Abbaurate aufweisen, die unter der Abbaurate des zentralen Deflektors (5) liegt.

3. Vaskuläres Implantat nach einem der vorangehenden Ansprüche, **dadurch gekennzeichnet, dass** die Mittel zum Halten des Deflektors aus einer Reihe von zickzackförmigen Abschnitten (1) bestehen, die untereinander durch Brücken (2) verbunden sind; und dadurch, dass zumindest zwei Haltearme (3), die an einem ihrer Enden mit einem zickzackförmigen Abschnitt (1) fest verbunden, aber an ihrem entgegengesetzten Ende frei sind, untereinander an ihren freien Enden durch einen Tropfen (4) aus bioresorbierbarem Polymer verbunden sind.

4. Vaskuläres Implantat nach einem der vorangehenden Ansprüche, **dadurch gekennzeichnet, dass** es gewonnen wird, indem ein Hohlzylinder aus ausscheidungshärtendem Material so ausgeschnitten wird, dass zumindest zwei Reihen von zickzackförmigen Abschnitten (1) und zumindest zwei Haltearme (3) des Deflektors (5) herausgelöst werden, und dadurch, dass das so ausgeschnittene Element an seinem grössten Durchmesser mit Hilfe eines Verformungswerkzeugs geöffnet und dann einer Hitzebehandlung unterworfen wird, durch die seine Gestalt fixiert werden kann.

5. Vaskuläres Implantat nach einem der Ansprüche 1 bis 3, **dadurch gekennzeichnet, dass** es gewonnen wird, indem ein Hohlzylinder aus ausscheidungshärtendem Material, der einen Durchmesser aufweist, der merklich über dem des Gefässes liegt, in das er eingeführt werden soll, so ausgeschnitten wird, dass zumindest zwei Reihen von zickzackförmigen Abschnitten (1) und zumindest zwei Haltearme (3) des Deflektors (5) herausgelöst werden, und dadurch, dass das so ausgeschnittene Element einer Hitzebehandlung unterworfen wird, durch die seine Gestalt fixiert werden kann.

6. Implantat nach einem der Ansprüche 1 bis 3, **dadurch gekennzeichnet, dass** die Haltemittel (1, 2, 3) des zentralen Deflektors (5) aus einem Formgedächtniswerkstoff gefertigt werden.

## Claims

1. A vascular implant comprising a flow deflector (5) of bio-resorbable material enabling flow lines to be deflected radially towards the arterial walls, and holding means (3) for holding the deflector (5) in the center of the vascular duct, the implant being **characterized by** the fact that the deflector holding means are prestressed to the center of the cylindrical structure and connected together by means that are bio-resorbable.

2. A vascular implant according to claim 1, **characterized by** the fact that the bio-resorbable means connecting the means for holding the deflector (5) presents a speed of degradation that is slower than the speed of degradation of the central deflector (5).

3. A vascular implant according to either preceding claim, **characterized by** the fact that the deflector holding means are constituted by a series of zigzag segments (1) interconnected by bridges (2); by the fact that at least two holding arms (3), each secured at one end to a zigzag segment (1) and each free at its opposite end, are interconnected via their free ends by means of a drop (4) of bio-resorbable polymer.

4. A vascular implant according to any preceding claim, **characterized by** the fact that it is obtained by being cut out from a hollow cylinder of material of settable structure so as to disengage at least two series of zigzag segments (1) and at least two arms (3) for holding the deflector (5), and by the fact that the element as cut out in this way is opened to its maximum diameter by means of a forming tool and is then subjected to heat treatment enabling its shape to be fixed.

5. A vascular implant according to any one of claims 1 to 3, **characterized by** the fact that it is obtained by being cut out from a hollow cylinder of material of settable structure presenting a diameter that is substantially greater than the diameter of the vessel into which it is be inserted, so as to disengage at least two series of zigzag segments (1) and at least two arms (3) for holding the deflector (5), and by the fact that the element as cut out in this way is subjected to heat treatment enabling its shape to be fixed.

6. An implant according to any one of claims 1 to 3, **characterized by** the fact that the means (1, 2, 3) for holding the central deflector (5) are made of a material having shape memory.
